# EUROPEAN PATENT SPECIFICATION

(11) **EP 2 595 561 B1**
(45) Date of publication and mention of the grant of the patent: **28.06.2017**
(21) Application number: 11738272.1
(22) Date of filing: 21.07.2011
(51) Int. Cl.: A61B 90/70

(54) **CLEANING AND/OR DISINFECTING APPARATUS**
REINIGUNGS- UND/ODER DESINFEKTIONSVORRICHTUNG
APPAREIL NETTOYANT ET/OU DÉSINFECTANT

(30) Priority: 21.07.2010 GB 201012213
(43) Date of publication of application: 29.05.2013
(73) Proprietor: Mantra Medical Limited, Sheffield, South Yorkshire S3 7SJ (GB)
(72) Inventor: HERCOCK, Paul Martin, Sheffield South Yorkshire S3 7SJ (GB); FREWER, Neil, Sheffield S60 5WF (GB); JONES, Richard Anthony Barritt, Sheffield South Yorkshire S1 4SA (GB)
(74) Representative: Foot, Paul Matthew James
(86) International application number: PCT/GB2011/051387
(87) International publication number: WO 2012/010902

(56) References cited:
- WO-A1-2007/085877
- WO-A2-2008/100950
- US-A- 3 556 667
- US-A- 5 865 551
- US-A- 6 018 835
- US-A1- 2006 213 920
- US-A1- 2010 116 841

## Description

The present invention relates to an apparatus for cleaning and/or disinfecting medical devices such as stethoscopes and pulse oximeters, and to a cartridge for such an apparatus. The present invention further relates to a method of using such an apparatus.

The medical stethoscope, for example, is used by virtually all medical doctors and many other healthcare professionals. Studies have shown that the stethoscope diaphragm (the part that usually comes into contact with the patient) is often colonised with large numbers of harmful bacteria including MRSA and *C.difficile.* Accordingly, the diaphragm may act as a source of transmission of potentially serious infections between patients causing significant morbidity and mortality.

Presently, UK hospitals advise that doctors should clean the diaphragm between patients using hand-held alcohol-soaked swabs, but this method is time consuming, cumbersome, and of questionable effectiveness. In practice this often means that the diaphragm is simply not cleaned at all. Similar advice is given regarding devices such as pulse oximeters.

What is required is an expedient and effective method to prevent the spread of infection between patients by such devices. It would be most effective if such a method was available in the same place as hand washing facilities so that all relevant cleaning could take place simultaneously. Crucially, any device performing such a function must be designed so that it does not itself become a source of infection, and being intended for use in a public health environment ease of maintenance is vitally important.

Document US-A-6 018 835 discloses an apparatus for cleaning a stethoscope according to the preamble of claim 1.

According to a first aspect of the present invention there is provided an apparatus for cleaning and/or disinfecting a medical device such as a stethoscope comprising a housing and a replaceable cartridge configured to fit within the housing. The cartridge contains cleaning medium for cleaning said medical device; and the apparatus defines an aperture configured to expose a surface of the medium to allow said medical device to be brought against said surface for cleaning. The medium is configured to advance with respect to the aperture such that different portions of the medium are exposed at the aperture.

The exposed surface of the medium carries cleaning fluid, and the cartridge includes a closed reservoir of cleaning fluid configured to be expressed onto the medium such that the exposed surface of the medium carries cleaning fluid. The apparatus has a mechanism to express cleaning fluid onto the medium. The housing may include a first actuator configured to express cleaning fluid from the reservoir. Alternatively, the medium may be pre-impregnated with cleaning fluid. The apparatus may include a perforated plate between the reservoir and the medium, the plate being configured to disperse the fluid onto the medium. The cleaning fluid may be a disinfectant fluid, preferably alcohol.

The housing may include a second, drive actuator arranged to advance the medium with respect to the aperture such that different portions of the medium are exposed at the aperture. The cartridge may include a first reel upon which the medium is supported prior to exposure at the aperture, and/or a second reel upon which medium that has been exposed at the aperture is collected. The first reel may be located at a first end of the housing and the second reel may be located at a second end of the housing, wherein the second actuator may be located between the first and second reels. The second actuator may be configured to engage and rotate the second reel such that the medium is advanced with respect to the aperture.

The apparatus may include a sensor configured to detect a medical device being brought against the exposed surface of the medium for cleaning. The second actuator may be configured to operate a predetermined period of time after detection of a medical device being brought against the exposed surface of the medium for cleaning.

The housing may include a third actuator arranged to agitate the medium at the exposed surface. The third actuator may be configured to operate upon detection of a medical device being brought against the exposed surface of the medium for cleaning.

The cleaning medium may be in the form of a continuous web, and/or may be a non-woven fabric, preferably air-laid paper.

The housing may include a shutter arrangement configured to close the aperture, and/or may have a door configured to allow removal and replacement of the cartridge. The apparatus may be configured for attachment to a substantially vertical surface such as a wall.

There is further provided a cartridge for use with an apparatus of the first aspect of the present invention for cleaning a medical device such as a stethoscope, the cartridge comprising a first reel upon which is supported cleaning medium for cleaning said medical device, a second reel and a closed reservoir of cleaning fluid configured to be expressed onto the medium such that the exposed surface of the medium carries the cleaning fluid when in use. The cartridge may further comprise a cartridge housing within which the first and second reels are supported.

There is yet further provided a method of cleaning a medical device such as a stethoscope comprising the steps of:
a) providing an apparatus as described above;
b) exposing a fresh surface of medium at the aperture;
c) holding the medical device against the exposed surface; and
d) agitating the exposed surface of the medium.

An example of the invention will now be described with reference to the accompanying drawings in which:
Figure 1 is a front perspective view of the workings of an example device with certain components omitted for clarity;
Figure 2 is a cross-sectional side view detailing how the cartridge containing the supply of cleaning surface interfaces with the mechanism shown in Fig 1;
Figure 3 is a cross-sectional side view that shows the detail of the cleaning ribbon of the first embodiment;
Figure 4 is a plan view of a cleaning apparatus according to a first embodiment of the present invention, with the door of the housing in an open position;
Figure 5 is a perspective view of the cleaning apparatus of Figure 4, with the housing door in a closed position;
Figure 6 is an exploded view of the cartridge and housing of the cleaning apparatus of Figures 4 and 5;
Figure 7 is a side view of a cartridge of the cleaning apparatus of Figures 4 to 6;
Figure 8 is a perspective view of the housing of the cleaning apparatus of Figures 4 to 7 with the door removed;
Figure 9 is a schematic view of a control system of the apparatus.

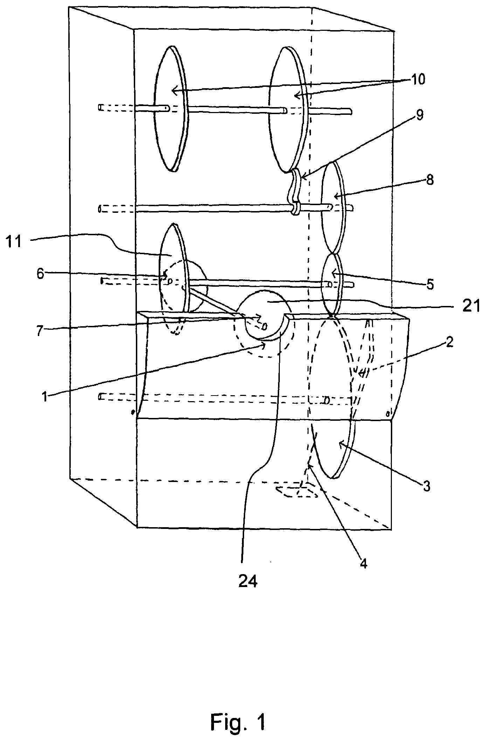

With reference to the example as shown in Figures 1 to 3, a stethoscope (not shown) is brought into contact with a device at contact plate 1. Depicted schematically in Fig 1, the contact plate 1 will form part of the aesthetics of the unit. The contact plate 1 has a groove 24 designed to accommodate the metal rod that projects from the inferior pole of every stethoscope diaphragm to the adjoining flexible tube that ultimately travels to the earpieces. This metal rod does not come into contact with the patient, but conveniently provides the rigidity necessary to initiate the movement required by the device. The metal rod is common to all stethoscopes.

Contact plate 1 is moved forwards by pressure from the user. Via a connecting rod 2 this forward motion partially rotates a driving cog 3. An elastic restraint 4, extending between the driving cog 3 and the base of the unit, returns the driving cog 3 to its original position at the end of the cleaning process.

The driving cog 3 interfaces with an amplifying cog 5. The amplifying cog 5 is significantly smaller than the driving cog 3, meaning that the degree of rotary movement initiated by the forwards motion of the contact plate 1 and transformed by the driving cog 3 is significantly amplified. The plane of rotation of the amplifying cog 5 is the same as that of the driving cog 3, e.g. as depicted in Fig 1.

The motion of the amplifying cog 5 is transferred via a rod 21 to a translating amplifier 6. The translating amplifier 6 rotates in the same plane as the amplifying cog 5, but interfaces with a smaller translator 11 at a 90 degree angle. Therefore, the translation amplifier 6 further amplifies the degree of rotation while the translator 11 simultaneously alters the plane of movement by 90 degrees.

This movement is then transmitted to a cleaning disc 7. In operation, the cleaning disc 7 will be separated from the diaphragm of the stethoscope by a cleaning ribbon 13, the specifics of which are detailed later. On initial forwards movement of the stethoscope and the contact plate 1, the cleaning disc 7 rapidly rotates in one direction. As the restraint 4 is fully stretched the forwards movement initiated by the user is halted; as the user releases pressure on the contact plate 1 the restraint 4 pulls the driving cog 3 back to its original position, operating the mechanism detailed above in reverse and producing rapid counter rotation of the cleaning disc 7 while the diaphragm of the stethoscope is still in contact with it, via the cleaning ribbon 13.

Simultaneously, the amplifying cog 5 interfaces with the diminishing cog 8. The diminishing cog 8 is larger than the amplifying cog 5, thereby diminishing the degree of rotation. It rotates in the same plane as the amplifying cog 5. The bi-directional movement of the mechanism is converted to unidirectional movement as a ratchet 9 interfaces with a cartridge driver 10. This ensures that while the cleaning disc 7 rotates in two directions, a cartridge 25 containing the cleaning ribbon 13 moves only in the required single direction. In construction, the exact degree of cartridge movement may be tuned by altering the size difference between the amplifying cog 5 and the diminishing cog 8.

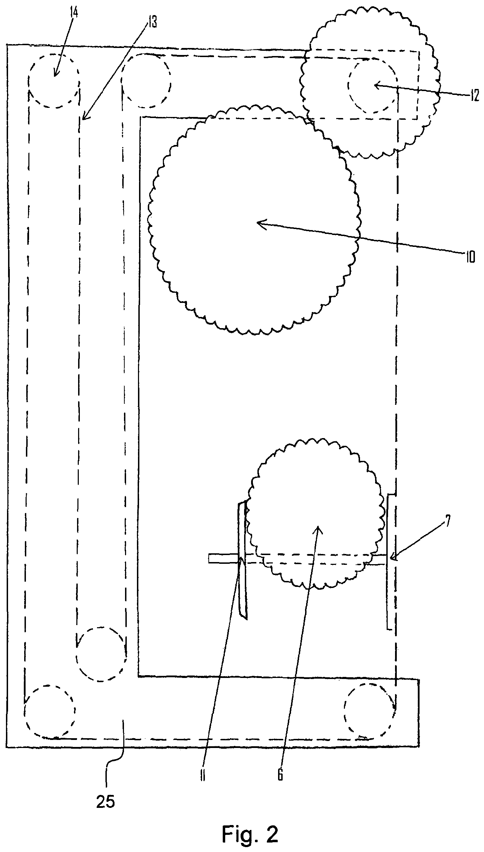

As depicted in Fig 2, the cartridge driver 10 interfaces with a ribbon cog 12 to drive movement of the cleaning ribbon 13 contained within the cartridge. The cleaning ribbon 13 is housed within the cartridge by spooling around spools 14, which may produce vertical folding of the ribbon 13 e.g. as depicted in Fig 2 or alternatively by folding the cleaning ribbon 13 horizontally.

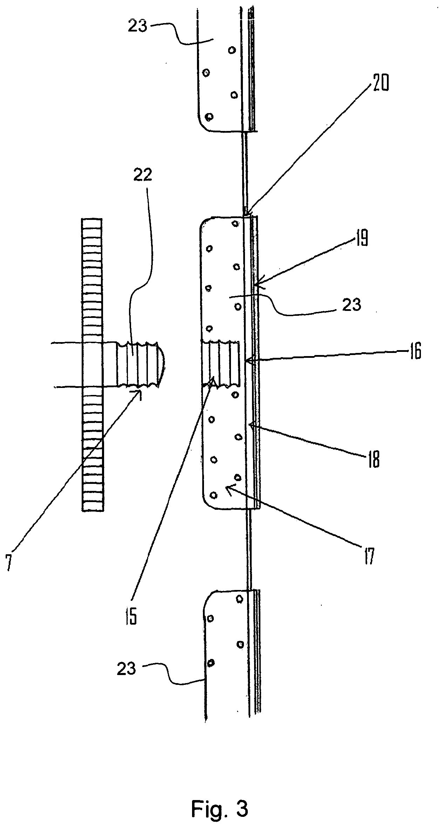

In Fig 3 the detail of the cleaning ribbon 13 may be observed. As depicted, it is composed of several 'bubbles' 23 separated by connecting areas. The cleaning disc 7 has a short, blunt ended screw bit 22 projecting anteriorly; during operation this screw bit 22 interfaces with a receptacle 15 as depicted. On initial rotation of the cleaning disc 7 the cleaning bubble 23 is rotated likewise; on subsequent counter-rotation friction between the body of the cleaning disc 7 and the posterior of the cleaning bubble 23 produces similar rotation of the bubble 23 until the screw bit 22 disengages from the receptacle 15, at which point the bubble 23 is released. At initiation of rotation the bubble 23 disengages from the body of the cleaning ribbon 13 at perforations 20, which pass circumferentially around the bubble 23 at a 90 degree angle to the plane of Fig 3.

Pressure from the screw bit 22 causes opening of a valve 16, allowing cleaning fluid stored within the reservoir 17 to flow into a potential space 18. This soaks a cleaning surface 19 with cleaning fluid (e.g. alcohol fluid such as ethanol or isopropanol, or ethoanol mixed with dodecanoic acid). This provides chemical cleaning and couples with rotation to produce dual-action cleaning. Once the cycle is completed, the bubble 23 falls into a receptacle (not shown) and the mechanism is reset via action of the elastic restraint 4.

Each cartridge contains multiple cleaning bubbles 23. Once fully spent, the cartridge is removed from a door (not shown) in the side of the unit and replaced.

In an alternative version (not shown), the cleaning disc 7 and the cartridge driver 10 are each moved by an electric motor that replaces the amplification and diminishing aspects of the mechanism detailed above, and which is initiated by a variety of means - in one version by depression of a switch or sensor mounted within the contact plate 1, in another by use of an infrared detector, and in another by a combination of means. Hence, when the user approaches the unit with a stethoscope, the same metal rod within the construction of the stethoscope initiates cleaning activity within the unit. In this motorised version the cleaning disc 7 may vibrate or agitate in a way other than a rotator action as detailed above, or alternatively it may rotate as above.

In another alternative version (not shown), the cleaning ribbon 13 may be a simple roll of fabric or paper, and may receive alcohol or other cleaning fluid from a separate reservoir within the device.

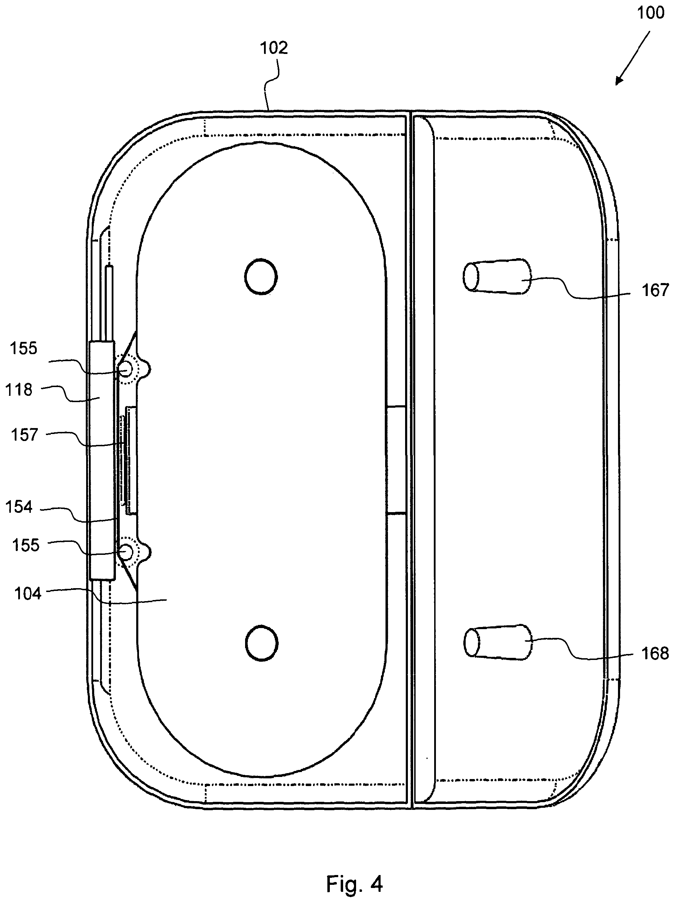

A second embodiment of the present invention shown in Figures 4 to 9 is an example of the version described in the preceding paragraph. An apparatus for cleaning medical devices such as stethoscopes is indicated generally at 100. The apparatus 100 has a housing 102 that defines a cavity 103, within which is contained a replaceable cartridge 104. The cartridge 104 contains cleaning media 136 against which medical devices can be cleaned, utilising similar principles to the first embodiment.

The cartridge 104 contains consumable material that will be used up during operation of the apparatus 100. The housing 102 contains actuating means for controlling operation of the apparatus 100. Various parts of the housing 102 engage parts of the cartridge 104 upon fitting of the cartridge 104, and operate the cartridge 104 through this engagement (see below). Once the consumable material within the cartridge 104 has been finished, the cartridge 104 is removed, and replaced with a new cartridge 104.

In this embodiment, the housing 102 is generally cuboid, with rounded edges, and has a front face 106, an upper end 108, a lower end 110 and sides 112. One of the sides 112 comprises a hinged door 114, which may be opened primarily to allow access to the cartridge cavity 103 for removal of the cartridge 104.

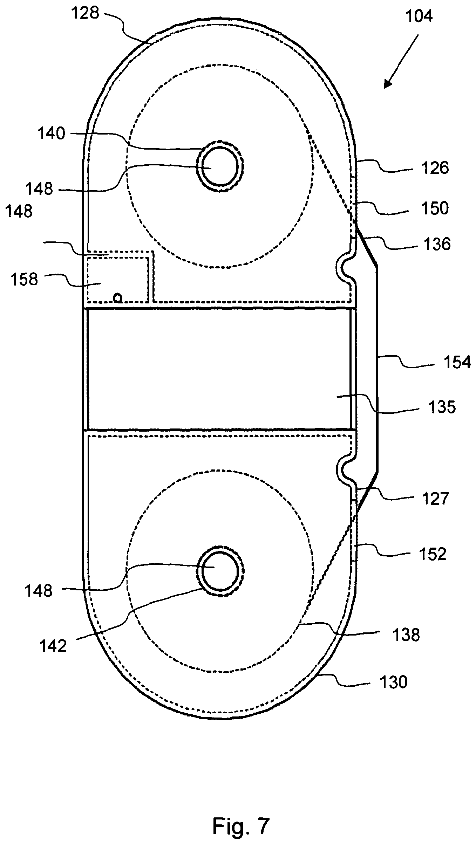

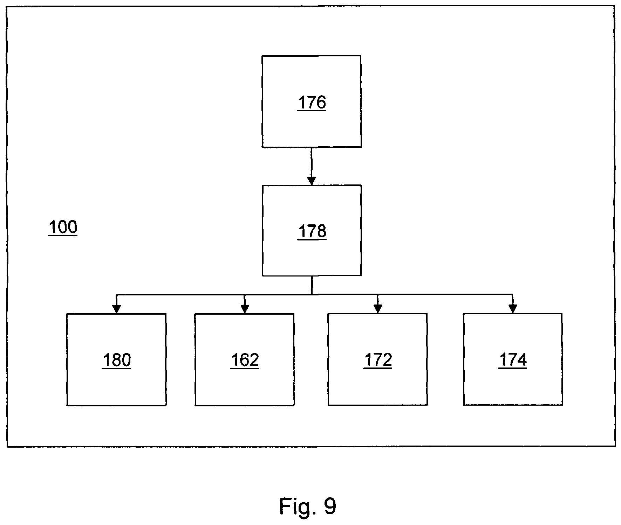

The front face 106 defines a circular aperture 116 which provides access to the cartridge 104 for a medical device to be cleaned. The housing 102 includes a shutter arrangement 118 on the inside of the front face 106 (e.g. as shown in Figure 7) that may be used to close the aperture 116. The shutter arrangement 118 has a grooved frame 120 that defines an aperture 122 which corresponds to the housing aperture 116, and a shutter 124 that is held within the frame 120. The shutter 124 is movable between a fully open position where the aperture 116 is accessible, and a closed position, where the aperture 116 is fully closed. The shutter 124 is shown in Figure 4 in a partially open position. The shutter 124 is moved between these positions by a motor 180 (see Figure 9) or other actuator. In alternative embodiments the shutter 124 may be moved manually. When closed, the shutter arrangement 118 inhibits the ingress of contaminants into the apparatus 100. In alternative embodiments there is no shutter arrangement.

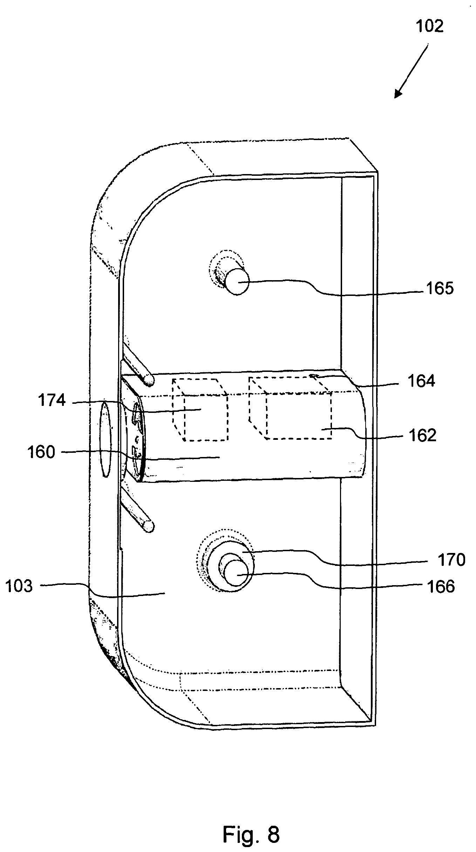

The housing 102 further has a protruding central portion 160 within the cavity 103 (e.g. as shown in Figure 8) that includes various actuators used to operate the apparatus 100, as well as a controller for controlling operation.

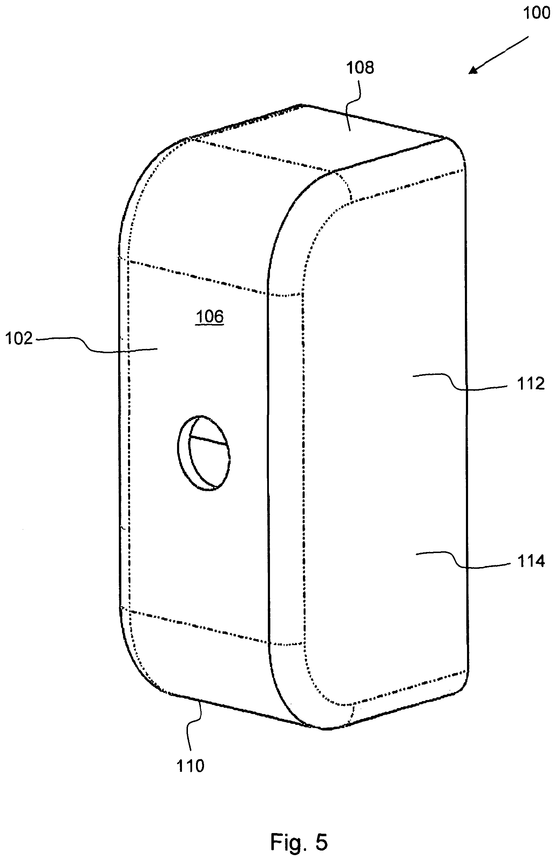

Figure 5 shows the housing 102 with the door 114 in an open position, displaying the cartridge 104 in position within the housing 102. The cartridge 104 is shown without the housing 102 in Figure 7. Internal components of the cartridge 104 are shown in broken lines.

The cartridge 104 has a cartridge housing 126 having a front face 127, a rounded upper end 128, a rounded lower end 130 and parallel first 132 and second 134 sides. The cartridge housing 126 defines a recess 135 in the first side 132. The recess 135 corresponds to the central portion 160, which extends into the recess 135 when the cartridge 104 is fitted within the housing 102. The actuators within the central portion 160 engage with the components of the cartridge 104, as will be described in further detail below.

The housing 126 contains the cleaning medium 136. In this embodiment, the cleaning medium 136 is in the form of a web of material 138, preferably non-woven material such as air-laid paper, or some other paper or fabric capable of fluid retention.

The web 138 is initially wound around a first reel 140. An end (not shown) of the web 138 extending from the first reel 140 is attached to a second reel 142, so that winding the second reel 142 results in the web 138 being gradually transferred from the first reel 140 to the second reel 142. The reels 140, 142 are rotatably supported by respective tubular, open ended axles 144, 146 extending between the parallel sides 132, 134. The first reel 140 is at the upper end 128, supported by the first axle 144, while the second reel 142 is at the lower end 130, supported by the second axle 146. Each axle 144, 146 defines a bore 148 extending through the cartridge housing 126.

The web 138 is arranged in the cartridge 104 to pass from the first reel 140 and through an upper slot 150 defined by the front face 127 of the housing 126 to the exterior of the cartridge 104. The web 138 returns to the interior of the cartridge 104 through a lower slot 152 defined by the front face 127, extending to the second reel 142. The surface of a portion 154 of the web 138 is thus exposed. When the cartridge 104 is fitted within the housing 102 as shown in Figure 4 the surface 154 is accessible to a user through the aperture 116. The surface 154 is stretched over two pins 155 that extend across the cavity 103 parallel to the front face 106, and is backed by a backing plate 157. In use, a user holds a stethoscope diaphragm to the surface 154 against the backing plate 157.

The cartridge 104 contains a reservoir 156 holding a cleaning, preferably disinfectant, fluid 158 (e.g. alcohol fluid such as ethanol or isopropanol, or ethoanol mixed with dodecanoic acid). The fluid 158 is dispersed over the surface 154 immediately prior to use for improved cleaning by a first actuator 162 (see Figure 9) within the central portion 160 of the housing 102. In this embodiment the first actuator is a peristaltic pump 162 that acts on a flexible pipe (not shown) that extends between the reservoir 156 and the surface 154, connecting with the reservoir 156 through an aperture 164 on the upper side of the central portion 160 and a corresponding aperture (not shown) in the reservoir 156. Advantageously, the fluid 158 does not therefore contact the moving parts of the pump 162. In alternative embodiments, other types of pump or other actuators may be used. The reservoir 156 may have a pierceable membrane or a valve to prevent leakage of the fluid 158 that is opened by a suitable feature of the housing 102, e.g. a rim of the aperture 164, upon fitting of the cartridge 104.

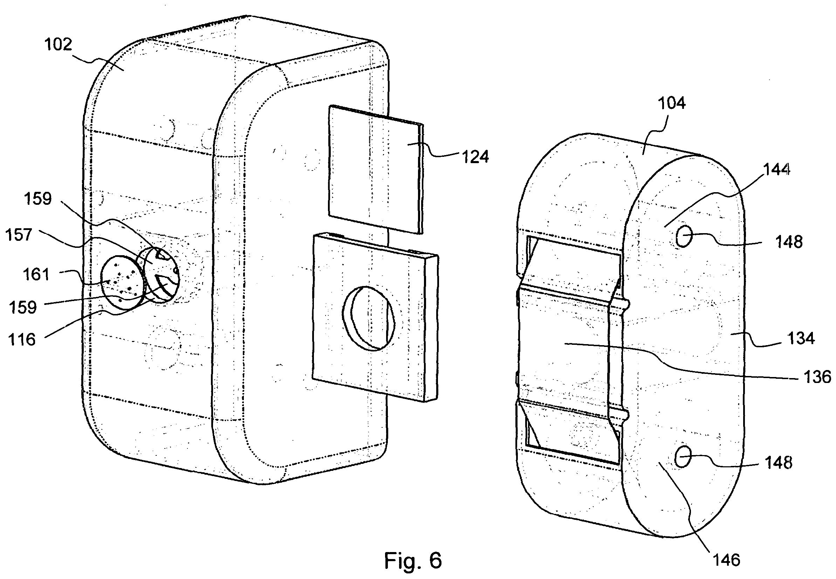

Upon operation of the pump 162 a predetermined amount of fluid 158 is expressed onto the surface 154 via a conduit (not shown) and through apertures 159 in the backing plate 157. Fluid 158 is dispersed onto the web 138 through a diffusing plate 161 (see Figure 6) which diffuses the fluid 158 so that an increased surface area of fluid 158 is provided for cleaning. The web 138 absorbs the fluid 158 so that it seeps through to the exposed surface 154. Means for chemical cleaning, or disinfecting, of the stethoscope is thus provided.

In alternative embodiments, the web 138 is pre-impregnated with cleaning fluid, and no reservoir is provided.

The cartridge 104 is supported within the housing 102 by upper 165 and lower 166 locating pins (see Figure 8), which extend within the cavity 103 proximal and parallel to the front face 106. The pins 165, 166 extend part way through the bores 148. The door 114 comprises further locating pins 167, 168 (see Figure 4), which extend part way through the bores 148 when the door 114 is closed, further supporting the cartridge 104.

The lower pin 166 has at its base a housing 170 for a second actuator, a drive motor 172 (see Figure 9) arranged to extend through the pin 166 to drive the second reel 142, thereby spooling the web 138 from the first reel 140 to the second reel 142. Turning of the first reel 140 on the axle 144 is frictionally or resiliently inhibited in order to keep the web 138 taut between the reels 140, 142, for example by biasing of the reel 140 against the cartridge housing 126. In alternative embodiments, other actuators may be used.

The central portion 160 further includes a third actuator 174 (see Figure 9) configured to agitate the backing plate 157 so that the surface 154 is agitated, in order to facilitate mechanical cleaning of the stethoscope. In this embodiment the third actuator 174 is a motor with an eccentric drive that oscillates the backing plate 157 via a cam and follower. In alternative embodiments the third actuator 174 may be a linear motor, or other suitable types of actuator.

The central portion 160 includes in this embodiment a battery (not shown) used to power the actuators 162, 172, 174, sensor 176 and control system 178. In alternative embodiments other power sources may be used.

The apparatus 100 includes a sensor 176 (see Figure 9) configured to detect the presence of a stethoscope at the cleaning surface 154. In this embodiment, the sensor 176 detects pressure on the backing plate 157. In other embodiments, a light sensor or Hall effect sensor may be used to detect the presence of the stethoscope at the aperture 116, or some other aspect. The sensor 176 signals a control system 178 (e.g. a microprocessor controller suitably programmed) situated within the central portion 160. The control system 178 controls the actuators 162, 172, 174, so that, in use, the apparatus 100 operates as follows.

The user holds a stethoscope up to the apparatus 100. The shutter 124 is opened by the control system 160 via the motor 180, making the aperture 116 accessible. The shutter 124 may be opened following detection of the stethoscope by a sensor such as an infra red sensor in the housing 102. Alternatively a command may be given to the control system 116 to open the shutter 124, e.g. by pressing a button on the housing.

The user holds the stethoscope so that its diaphragm contacts the surface 154, pushing the surface 154 onto the backing plate 157. The sensor 176 detects the presence of the stethoscope. The control system 178 operates the pump 162 to supply fluid 158 to the surface 154, and the third actuator 174 to agitate the backing plate 157. Fluid 158 disperses through the diffusing plate 161 and soaks through the web 138 to the surface 154, and so to the diaphragm, disinfecting the diaphragm. Agitation of the backing plate 157 assists disinfection, and causes mechanical cleaning of the diaphragm, removing contaminants.

The third actuator 174 ceases to operate upon removal of the stethoscope from the surface 154. In alternative embodiments the third actuator 174 is operated for a predetermined length of time, based on the predicted time taken to clean the stethoscope.

Once cleaning of the stethoscope has taken place, the control system 178 activates the drive motor 172 to advance the web 138 so that a fresh portion of the web 138 is exposed at the aperture 116. Alternatively, a fresh portion of the web 138 may be exposed upon detection of a stethoscope prior to cleaning, or both prior to and after cleaning.

The housing 102 has a rear face including mounting points (not shown) whereby the housing 102 may be attached to a wall or other surface for convenience of use.

There are multiple advantages to the cleaning apparatus of this embodiment. The apparatus 100 provides quick, effective cleaning. Both mechanical and chemical cleaning are provided, and are carried out simultaneously. The apparatus 100 may be conveniently situated on a wall or other surface, for example near hand washing facilities or at an entrance to a ward.

The replaceable cartridge 104 allows the apparatus 100 to be replenished quickly and easily. The closed reservoir and the shutter arrangement inhibit the build up of germs on or in the apparatus 100 itself, reducing the possibility of the apparatus 100 becoming a source of infection.

In alternative embodiments (not shown) the web 138 may be advanced by manual activation of the second reel 142, e.g. by a handle extending from the housing.

Stethoscopes may be fitted with a radio frequency ID tag (RFID tag), barcode, QR code, or other suitable machine readable identifier and the device may be provided with a corresponding reader so that each stethoscope may be identified by the apparatus 100, and a log kept of stethoscopes cleaned by the apparatus 100.

The apparatus 100 may give an indication of when the web 138 is close to being finished, e.g. by flashing a light or emitting an alarm sound.

The apparatus 100 can be used to clean other medical devices, such as pulse oximeters, laryngoscope blades or specula, and may be adapted for use with such devices, e.g. to have a rotating or reciprocating protrusion in place of the backing plate 157 to correspond with a pulse oximeter.

## Claims

1. An apparatus (100) for cleaning and/or disinfecting a medical device such as a stethoscope or pulse oximeter comprising a housing (102) and a replaceable cartridge (104) configured to fit within the housing;
the cartridge containing cleaning medium (136) for cleaning said medical device; and the apparatus defining an aperture (116) configured to expose a surface of the medium (136) to allow said medical device to be brought against said surface for cleaning;
wherein the medium (136) is configured to advance with respect to the aperture such that different portions of the medium (136) are exposed at the aperture and the exposed surface of the medium carries cleaning fluid;
wherein the cartridge includes a reservoir (156) of cleaning fluid (158) configured to be expressed onto the medium such that the exposed surface of the medium carries cleaning fluid, wherein the reservoir is a closed reservoir,
**characterised in that** the housing has a first actuator (162) configured to express said cleaning fluid from the reservoir onto the medium.

2. An apparatus according to any preceding claim further including a diffusing plate (161) between the reservoir and the medium, the plate being configured to diffuse the fluid (158) onto the medium (136).

3. An apparatus according to any preceding claim wherein the housing includes a second, drive actuator (172) arranged to advance the medium (136) with respect to the aperture such that successive portions of the medium are exposed at the aperture.

4. An apparatus according to any preceding claim wherein the cartridge includes a first (140) reel upon which the medium (136) is supported prior to exposure at the aperture and/or the cartridge includes a second reel (142) upon which medium (136) that has been exposed at the aperture is collected.

5. An apparatus according to claim 4 when dependent upon claim 3 wherein the first reel (140) is located at a first end of the housing and the second reel (142) is located at a second end of the housing, and wherein the second actuator (172) is located between the first and second reels.

6. An apparatus according to claim 4 or claim 5 when dependent upon claim 3 wherein the second actuator (172) is configured to engage and rotate the second reel (142) such that the medium (136) is advanced with respect to the aperture.

7. An apparatus according to any preceding claim including a sensor (176) configured to detect a medical device being brought against the exposed surface of the medium for cleaning.

8. An apparatus according to claim 7 when dependent upon any one of claims 3 to 6 wherein the second actuator (172) is configured to operate a predetermined period of time after detection of a medical device being brought against the exposed surface of the medium (136) for cleaning.

9. An apparatus according to any preceding claim wherein the cleaning medium (136) is in the form of a continuous web.

10. An apparatus according to any preceding claim wherein the housing includes a shutter arrangement (118) configured to close the aperture (116).

11. An apparatus according to claim 1, wherein the cartridge comprises a first reel (140) upon which is supported said cleaning medium (136) for cleaning said medical device, a second reel (142).

12. A method of cleaning a medical device such as a stethoscope or pulse oximeter comprising the steps of:
a) providing an apparatus (100) according to any of claims 1 to 10;
b) exposing a fresh surface of the medium (136) at the aperture;
c) holding the medical device against the exposed surface; and
d) agitating the exposed surface of the medium.

## Patentansprüche

1. Vorrichtung (100) zum Reinigen und/oder Desinfizieren eines medizinischen Geräts wie beispielsweise eines Stethoskops oder Pulsoximeters, umfassend ein Gehäuse (102) und eine austauschbare Kassette (104), die derart konfiguriert ist, dass sie in das Gehäuse passt;
wobei die Kassette ein Reinigungsmedium (136) zum Reinigen des medizinischen Geräts enthält; und wobei die Vorrichtung eine Öffnung (116) definiert, die konfiguriert ist für das Exponieren einer Oberfläche des Mediums (136) zu, so dass das medizinische Gerät zum Reinigen an die Oberfläche herangebracht werden kann;
wobei das Medium (136) konfiguriert ist für eine Bewegung gegenüber der Öffnung, so dass verschiedene Abschnitte des Mediums (136) an der Öffnung exponiert werden, und wobei die exponierte Oberfläche des Mediums ein Reinigungsfluid trägt;
wobei die Kassette ein Reservoir (156) mit Reinigungsfluid (158) enthält, das dazu ausgebildet ist, auf das Medium ausgedrückt zu werden, so dass die exponierte Fläche Reinigungsfluid trägt; wobei das Reservoir ein geschlossenes Reservoir ist,
**dadurch gekennzeichnet, dass** das Gehäuse einen ersten Aktuator (162) aufweist, der zum Ausdrücken des Reinigungsfluides aus dem Reservoir auf das Medium ausgebildet ist.

2. Vorrichtung nach einem der vorhergehenden Ansprüche, ferner umfassend eine Diffusionsplatte (161) zwischen dem Reservoir und dem Medium, wobei die Platte konfiguriert ist für das Verteilen des Fluides (158) auf dem Medium (136).

3. Vorrichtung nach einem der vorhergehenden Ansprüche, wobei das Gehäuse einen zweiten, treibenden Aktuator (172) aufweist, der angeordnet ist zum Bewegen des Mediums (136) gegenüber der Öffnung, so dass aufeinanderfolgende Abschnitte des Mediums an der Öffnung exponiert werden.

4. Vorrichtung nach einem der vorhergehenden Ansprüche, wobei die Kassette eine erste Spule (140) enthält, auf welcher das Medium (136) getragen wird, bevor dieses an der Öffnung exponiert wird, und/oder wobei die Kassette eine zweite Spule (142) enthält, auf welcher das Medium (136), das an der Öffnung exponiert wurde, aufgenommen wird.

5. Vorrichtung nach Anspruch 4, wobei, wenn abhängig von Anspruch 3, die erste Spule (140) an einem ersten Ende des Gehäuses und die zweite Spule (142) an einem zweiten Ende des Gehäuses liegt und der zweite Aktuator (172) zwischen der ersten und der zweiten Spule liegt.

6. Vorrichtung nach Anspruch 4 oder Anspruch 5, wobei, wenn abhängig von Anspruch 3, der zweite Aktuator (172) konfiguriert ist für den Eingriff in die zweite Spule und für das Drehen der Spule (142), so dass das Medium (136) gegenüber der Öffnung weiterbewegt wird.

7. Vorrichtung nach einem der vorhergehenden Ansprüche, umfassend einen Sensor (176), der ausgebildet ist zum Detektieren eines medizinischen Geräts, das zum Reinigen an die exponierte Oberfläche des Mediums herangebracht wird.

8. Vorrichtung nach Anspruch 7, wobei, wenn abhängig von einem der Ansprüche 3 bis 6, der zweite Aktuator (172) konfiguriert ist für den Betrieb über eine vorgegebene Zeitspanne nach dem Detektieren eines medizinischen Geräts, das zum Reinigen an die exponierte Oberfläche des Mediums (136) herangebracht wurde.

9. Vorrichtung nach einem der vorhergehenden Ansprüche, wobei das Reinigungsmedium (136) eine Endlosbahn ist.

10. Vorrichtung nach einem der vorhergehenden Ansprüche, wobei das Gehäuse eine Verschlussanordnung (118) aufweist, die zum Verschließen der Öffnung (116) ausgebildet ist.

11. Vorrichtung nach Anspruch 1, wobei die Kassette eine erste Spule (140) aufweist, auf welcher das Reinigungsmedium (136) zum Reinigen des medizinischen Geräts getragen ist, und eine zweite Spule (142).

12. Verfahren zum Reinigen eines medizinischen Geräts wie beispielsweise eines Stethoskops oder eines Pulsoximeters, umfassend die folgenden Schritte:
a) Bereitstellen einer Vorrichtung (100) gemäß einem der Ansprüche 1 bis 10;
b) Exponieren einer frischen Oberfläche des Mediums (136) an der Öffnung;
c) Halten des medizinischen Geräts an die exponierte Oberfläche; und
d) Bewegen der exponierten Oberfläche des Mediums.

## Revendications

1. Appareil (100) pour nettoyer et/ou désinfecter un dispositif médical tel qu'un stéthoscope ou un sphygmo-oxymètre comprenant un logement (102) et une cartouche remplaçable (104) configurée pour s'ajuster dans le logement ;
la cartouche contenant un milieu nettoyant (136) pour nettoyer ledit dispositif médical ; et l'appareil définissant une ouverture (116) configurée pour exposer une surface du milieu (136) pour permettre audit dispositif médical d'être placé contre ladite surface pour le nettoyage ;
dans lequel le milieu (136) est configuré pour avancer par rapport à l'ouverture de sorte que différentes portions du milieu (136) soient exposées à l'ouverture et la surface exposée du milieu porte le fluide nettoyant ;
dans lequel la cartouche comprend un réservoir (156) pour nettoyer un fluide (158) configuré pour être exprimé sur le milieu de sorte que la surface exposée du milieu porte le fluide nettoyant, dans lequel le réservoir est un réservoir fermé,
**caractérisé en ce que** le logement a un premier actionneur (162) configuré pour exprimer ledit fluide nettoyant du réservoir sur le milieu.

2. Appareil selon l'une quelconque des revendications précédentes, comportant en outre une plaque de diffusion (161) entre le réservoir et le milieu, la plaque étant configurée pour diffuser le fluide (158) sur le milieu (136).

3. Appareil selon l'une quelconque des revendications précédentes, dans lequel le logement comporte un deuxième actionneur d'entraînement (172) agencé pour faire avancer le milieu (136) par rapport à l'ouverture de sorte que des portions successives du milieu soient exposées sur l'ouverture.

4. Appareil selon l'une quelconque des revendications précédentes, dans lequel la cartouche comporte une première bobine (140) sur laquelle le milieu (136) est supporté avant une exposition à l'ouverture et/ou la cartouche comporte une deuxième bobine (142) sur laquelle le milieu (136) qui a été exposé sur l'ouverture est recueilli.

5. Appareil selon la revendication 4, lorsqu'elle est dépendante de la revendication 3, dans lequel la première bobine (140) est située sur une première extrémité du logement et la deuxième bobine (142) est située sur une deuxième extrémité du logement, et dans lequel ledit deuxième actionneur (172) est situé entre les première et deuxième bobines.

6. Appareil selon la revendication 4 ou la revendication 5, lorsqu'elles sont dépendantes de la revendication 3, dans lequel le deuxième actionneur (172) est configuré pour mettre en prise et faire tourner la deuxième bobine (142) de sorte que le milieu (136) soit avancé par rapport à l'ouverture.

7. Appareil selon l'une quelconque des revendications précédentes, comportant un capteur (176) configuré pour détecter un dispositif médical qui est placé contre la surface exposée du milieu pour le nettoyage.

8. Appareil selon la revendication 7, lorsqu'elle est dépendante de l'une quelconque des revendications 3 à 6, dans lequel le deuxième actionneur (172) est configuré pour fonctionner pendant une durée prédéterminée après une détection d'un dispositif médical qui est placé contre la surface exposée du milieu (136) pour le nettoyage.

9. Appareil selon l'une quelconque des revendications précédentes, dans lequel le milieu nettoyant (136) se présente sous la forme d'une toile continue.

10. Appareil selon l'une quelconque des revendications précédentes, dans lequel le logement comporte un agencement d'obturateur (118) configuré pour fermer l'ouverture (116).

11. Appareil selon la revendication 1, dans lequel la cartouche comprend une première bobine (140) sur laquelle est supporté ledit milieu nettoyant (136) pour nettoyer ledit dispositif médical, une deuxième bobine (142).

12. Procédé de nettoyage d'un dispositif médical tel qu'un stéthoscope ou un sphygmo-oxymètre comprenant les étapes de :
a) fourniture d'un appareil (100) selon l'une quelconque des revendications 1 à 10 ;
b) exposition d'une surface fraîche du milieu (136) sur l'ouverture ;
c) maintien du dispositif médical contre la surface exposée ; et
d) agitation de la surface exposée du milieu.
